# EUROPEAN PATENT SPECIFICATION

(11) **EP 3 383 461 B1**
(45) Date of publication and mention of the grant of the patent: **29.07.2020**
(21) Application number: 16805093.8
(22) Date of filing: 30.11.2016
(51) Int. Cl.: A61M 15/06, A61M 11/04, A24F 47/00

(54) **NON-COMBUSTIBLE SMOKING DEVICE AND ELEMENTS THEREOF**
NICHTBRENNBARE RAUCHVORRICHTUNG UND ELEMENTE DAVON
DISPOSITIF À FUMER NON COMBUSTIBLE ET ÉLÉMENTS DE CELUI-CI

(30) Priority: 30.11.2015 US 201562260793 P
(43) Date of publication of application: 10.10.2018
(73) Proprietor: Philip Morris Products S.A., 2000 Neuchâtel (CH)
(72) Inventor: TUCKER, Christopher S., Midlothian, VA 23114 (US); SMITH, Barry S., Hopewell, VA 23860 (US); CADIEUX, Edmond J., Mechanicsville, VA 23116 (US); BENNETT, David, Tappahannock, VA 22560 (US)
(74) Representative: Dowling, Ian
(86) International application number: PCT/EP2016/079343
(87) International publication number: WO 2017/093357

(56) References cited:
- WO-A1-2014/116974
- WO-A1-2015/046385
- CA-A1- 2 925 645
- US-A- 5 505 214
- US-A1- 2008 092 912
- US-A1- 2014 060 554
- US-A1- 2014 166 029
- US-A1- 2014 202 472
- US-A1- 2014 366 898

## Description

The present invention relates generally to a non- combustible smoking device as defined by the amended claims.

Electronic vaping devices are used to vaporize a pre-vapor formulation into a vapor. These electronic vaping devices may be referred to as e-vaping devices. E-vaping devices include a heater, which vaporizes the pre-vapor formulation to produce the vapor. The e-vaping device may include several e-vaping elements including a power source, a cartridge or e-vaping tank including the heater and a reservoir capable of holding the pre-vapor formulation.

US 2008/092912 A1 describes a smoking article comprising a cigarette in combination with an electrically powered aerosol-generating device. In some embodiments the cigarette comprises tobacco and an aerosol-forming material. The device comprises a first heater for heating air entering the device and a second heater positioned adjacent the tobacco or adjacent the aerosol-forming material. In some embodiments the cigarette is in the form of a cartridge and an air passageway extends around the cartridge for conveying vapor to a mouthpiece.

US 2014/366898 A1 describes cartridges for use with a vaporization device, each cartridge comprising at least first and second compartments for separate containment of vaporizable materials. Each compartment is annular in shape and is provided with a wick and a heater coil positioned within a central airflow passage. The compartments may be provided in series or parallel.

WO 2014/116974 A1 describes methods and apparatus for using fragrance inserts to impart organoleptic properties to vapor generated from an aerosol forming cartridge. The fragrance inserts may contain tobacco extracts.

At least one example disclosed relates to a non-combustible smoking device. The non-combustible smoking device may have a heater that heats a pre-vapor formulation and may provide heat to a tobacco element that receives the vapor. More specifically, the non-combustible smoke device according to example embodiments exposes a vapor to a tobacco element, exposes a pre-vapor formulation to a tobacco element, or both.

The invention is defined as a non-combustible smoking element including a pre-vapor formulation reservoir element configured to contain a pre-vapor formulation material, a pre-vapor heating element coupled to the pre-vapor formulation reservoir element and configured to heat at least a portion of the pre-vapor formulation material into a vapor and provide the vapor to a channel, a tobacco heating element configured to heat at least a portion of tobacco and generate an aroma and a tobacco housing configured to contain the tobacco and provide the aroma to the channel, wherein the tobacco housing includes an outer housing extending in a longitudinal direction, an inner tube in the outer housing and extending in the longitudinal direction, a space between the outer housing and the inner tube defining a space to contain the tobacco, and a connecting piece at a first end of the tobacco housing, the connecting piece including at least one first air inlet to provide air to the space between the outer housing and the inner tube, and wherein the connecting piece includes a second air inlet to provide air within the inner tube.

In an example embodiment, the tobacco heating element includes a plurality of heaters in the tobacco housing.

In an example embodiment, the channel extends through the tobacco housing.

In an example embodiment, the plurality of heaters are upstream from the pre-vapor heating element.

In an example embodiment, the tobacco is arranged such that the aroma is delivered to the vapor through the channel upon an action by an adult vaper of the non-combustible smoking element.

In an example embodiment, the plurality of heaters are outside the channel and the pre-vapor heating element is in the channel.

In an example embodiment, the tobacco heating element is a coil and extends around the inner tube.

In an example embodiment, the tobacco heating element extends around the inner tube at an interval of between about 1 and about 2 millimeters.

In an example embodiment, the tobacco housing includes one of a low efficiency cellulose acetate (CA) filter, glass fiber filter, mesh screen and silicon gasket at a second end of the tobacco housing.

In an example embodiment, the tobacco heating element contacts the tobacco.

In an example embodiment, the pre-vapor formulation reservoir element and the tobacco housing are detachable.

In an example embodiment, the tobacco is tobacco plant material in any form.

In an example embodiment, the tobacco heating element is upstream from the pre-vapor heating element.

In an example embodiment, the tobacco heating element is downstream from the pre-vapor heating element.

The above and other features and advantages of example embodiments will become more apparent by describing in detail, example embodiments with reference to the attached drawings. The accompanying drawings are intended to depict example embodiments and should not be interpreted to limit the intended scope of the claims which define the scope of the invention. The accompanying drawings are not to be considered as drawn to scale unless explicitly noted.

FIG. 1 is a cross-sectional view of a non-combustible smoking device including a tobacco element, in accordance with an example.

FIG. 2A is a perspective view of a mouth-end insert for use with the non-combustible smoking device of FIG. 1, in accordance with an example embodiment;

FIG. 2B is a cross-sectional view along line B-B of the mouth-end insert of FIG. 2A, in accordance with an example embodiment;

FIG. 3 is a cross-sectional view of an embodiment wherein a non-combustible smoking device includes an air flow diverter, in accordance with an example.

FIG. 4 is an enlarged view of the air flow diverter of the non- combustible smoking device of FIG. 3, in accordance with an example.

FIG. 5 is a cross-sectional view of an embodiment wherein a non-combustible smoking device includes an air flow diverter, in accordance with an example.

FIG. 6 is a cross-sectional view along line A-A of the non-combustible smoking device of FIG. 6, in accordance with an example.

FIG. 7 is a cross-sectional view of an embodiment wherein a non-combustible smoking device includes an air flow diverter, in accordance with an example.

FIG. 8 is a cross-sectional view of a non-combustible smoking device and further including a sleeve assembly, in accordance with an example.

FIG. 9 is a cross-sectional view of a second embodiment of a mouth-end insert for use with a non-combustible smoking device, in accordance with an example embodiment;

FIG. 10 is an exploded view of the mouth-end insert of FIG. 9, in accordance with an example embodiment;

FIGS. 11A-11B illustrate examples of a non- combustible smoking device including a tobacco element;

FIG. 12 illustrates an example of a non-combustible smoking device;

FIGS. 13A-13B illustrate examples of a non-combustible smoking device including a tobacco element;

FIGS. 14A-B illustrate an example embodiment of a pre-vapor formulation supply reservoir;

FIGS. 15A-B illustrates an example embodiment of a non-combustible smoking device having a plurality heaters;

FIG. 16 illustrates a top view of a coiled heater shown in FIG. 15A;

FIG. 17 illustrates a top view of a cathode portion shown in FIG. 15A;

FIG. 18 illustrates a tobacco housing for a non-combustible smoking device according to an example embodiment;

FIG. 19 illustrates another example embodiment of a non-combustible smoking device having a plurality heaters;

FIG. 20 illustrates a flip top container for a non-combustible smoking device according to an example embodiment; and

FIG. 21 illustrates a flip top container for a non-combustible smoking device according to another example embodiment.

Some detailed example embodiments are disclosed herein. However, specific structural and functional details disclosed herein are merely representative for purposes of describing example embodiments. Example embodiments may, however, be embodied in many alternate forms and should not be construed as limited to only the embodiments set forth herein. Any example embodiments disclosed herein only constitute part of the invention insofar as they fall under the scope of the appended claims.

Accordingly, while example embodiments are capable of various modifications and alternative forms, embodiments thereof are shown by way of example in the drawings and will herein be described in detail. It should be understood, however, that there is no intent to limit example embodiments to the particular forms disclosed, but to the contrary, example embodiments are to cover all modifications, equivalents, and alternatives falling within the scope of example embodiments. Like numbers refer to like elements throughout the description of the figures.

It should be understood that when an element or layer is referred to as being "on," "connected to," "coupled to," or "covering" another element or layer, it may be directly on, connected to, coupled to, or covering the other element or layer or intervening elements or layers may be present. In contrast, when an element is referred to as being "directly on," "directly connected to," or "directly coupled to" another element or layer, there are no intervening elements or layers present. Like numbers refer to like elements throughout the specification.

It should be understood that, although the terms first, second, third, and so forth may be used herein to describe various elements, regions, layers or sections, these elements, regions, layers, or sections should not be limited by these terms. These terms are only used to distinguish one element, region, layer, or section from another element, region, layer, or section. Therefore, a first element, region, layer, or section discussed below could be termed a second element, region, layer, or section without departing from the teachings of example embodiments.

Spatially relative terms (for example, "beneath," "below," "lower," "above," "upper," and the like) may be used herein for ease of description to describe one element or feature's relationship to one or more other elements or features as illustrated in the figures. It should be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Therefore, the term "below" may encompass both an orientation of above and below. The device may be otherwise oriented (rotated 90 degrees or at other orientations) and the spatially relative descriptors used herein interpreted accordingly.

The terminology used herein is for the purpose of describing various embodiments only and is not intended to be limiting of example embodiments. As used herein, the singular forms "a," "an," and "the" are intended to include the plural forms as well, unless the context clearly indicates otherwise. It will be further understood that the terms "includes," "including," "comprises," and "comprising," when used in this specification, specify the presence of stated features, integers, steps, operations, elements, or elements, but do not preclude the presence or addition of one or more other features, integers, steps, operations, elements, elements, or groups thereof.

Example embodiments are described herein with reference to cross-sectional illustrations that are schematic illustrations of idealized embodiments (and intermediate structures) of example embodiments. As such, variations from the shapes of the illustrations as a result, for example, of manufacturing techniques or tolerances, are to be expected. Therefore, example embodiments should not be construed as limited to the shapes of regions illustrated herein but are to include deviations in shapes that result, for example, from manufacturing. Therefore, the regions illustrated in the figures are schematic in nature and their shapes are not intended to illustrate the actual shape of a region of a device and are not intended to limit the scope of example embodiments.

Unless otherwise defined, all terms (including technical and scientific terms) used herein have the same meaning as commonly understood by one of ordinary skill in the art to which example embodiments belong. It will be further understood that terms, including those defined in commonly used dictionaries, should be interpreted as having a meaning that is consistent with their meaning in the context of the relevant art and will not be interpreted in an idealized or overly formal sense unless expressly so defined herein.

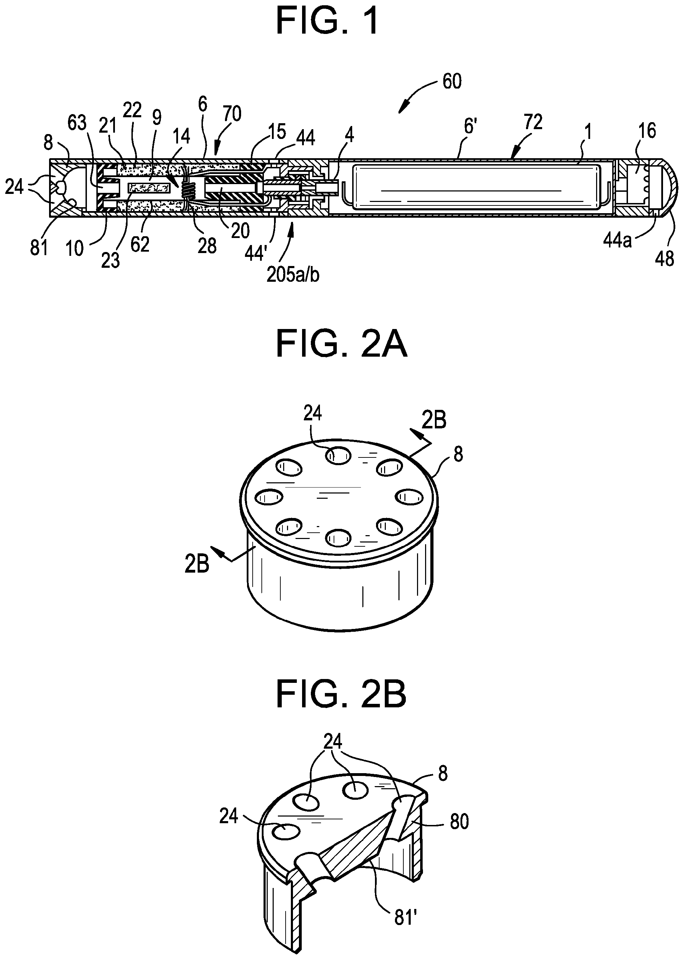

FIG. 1 illustrates a non-combustible smoking device 60 according to an example embodiment. The non-combustible smoking device 60 comprises a replaceable cartridge (or first section) 70 and a reusable fixture (or second section) 72, which are coupled together at a connection 205a and 205b (for example, 205a is a male threaded connection on cartridge 70, and 205b is a female threaded connection on reusable fixture 72) or by other convenience such as at least one of a snug-fit, detent, clamp or clasp. The first section 70 includes an outer tube 6 (or housing) extending in a longitudinal direction and an inner tube 62 coaxially positioned within the outer tube or housing 6. The inner tube 62 defines an outer air passage (or channel) 9. Within the outer air passage 9 and downstream from a heater 14 is a tobacco element 23. The tobacco element 23 may be in a porous aluminum tube, or processed or shaped in a porous form.

The term "tobacco element" may refer to any tobacco plant material including tobacco leaf, tobacco plug, reconstituted tobacco, compressed tobacco rod, shaped, or powder, for example.

The tobacco element 23 may also be wrapped in tobacco such as a tobacco sheet, a reconstituted tobacco leaf or a cigar wrapper.

The second section 72 can also include an outer tube 6' (or housing) extending in a longitudinal direction. In an alternative embodiment, the outer tube 6 and 6' can be a single tube housing both the first section 70 and the second section 72 and the entire non-combustible smoking device 60 can be disposable.

The non-combustible smoking device 60 can also include a central air passage 20 defined in part by the inner tube 62 and an upstream seal 15. Moreover, the non-combustible smoking device 60 includes a pre-vapor formulation supply reservoir 22. The pre-vapor formulation supply reservoir 22 comprises a pre-vapor formulation material and optionally a pre-vapor formulation storage medium 21 operable to store the pre-vapor formulation material therein.

In an embodiment, the pre-vapor formulation supply reservoir 22 is contained in an outer annulus between the outer tube 6 and the inner tube 62. The annulus is sealed at an upstream end by the seal 15 and by a pre-vapor formulation gasket 10 at a downstream end so as to prevent leakage of the pre-vapor formulation material from the pre-vapor formulation supply reservoir 22.

In an embodiment, a heater 14 is also contained in the inner tube 62 downstream of and in spaced apart relation to the portion of central air passage 20 defined by the seal 15. The heater 14 can be in the form of a wire coil, a planar body, a ceramic body, a single wire, a cage of resistive wire or any other suitable form.

A wick 28 is in communication with the pre-vapor formulation material in the pre-vapor formulation supply reservoir 22 and in communication with the heater 14 such that the wick 28 disposes pre-vapor formulation material in proximate relation to the heater 14. The wick 28 may be constructed of a fibrous and flexible material. The wick 28 may include at least one filament having a capacity to draw a pre-vapor formulation. For example, the wick 28 may comprise a bundle of filaments which may include glass (or ceramic) filaments. In another embodiment, a bundle comprising a group of windings of glass filaments, for example, three of such windings, all which arrangements are capable of drawing pre-vapor formulation via capillary action via interstitial spacing between the filaments.

A power supply 1 in the second section 72 may be operably connected to the heater 14 (as described below) to apply voltage across the heater 14. The non-combustible smoking device 60 also includes at least one air inlet 44 operable to deliver air to the central air passage 20, other portions of the inner tube 62, or both.

As shown in FIGS. 1-2B, the non-combustible smoking device 60 further includes a mouth-end insert 8 having at least two off-axis, diverging outlets 24. The mouth-end insert 8 is in fluid communication with the central air passage 20 via the interior of inner tube 62 and a central passage 63, which extends through the gasket 10.

Moreover, the heater 14 extends in a direction transverse to the longitudinal direction and heats the pre-vapor formulation material to a temperature sufficient to vaporize the pre-vapor formulation material and form a vapor. In other embodiments, the heater 14 may be arranged in another manner such as in the longitudinal direction.

The vapor then flows into the tobacco element 23 upon an applying a negative pressure on the mouth-end insert 8. The heater 14 may be a set distance from the tobacco element 23 or contacting the tobacco element 23 such that the heater 14 heats the tobacco element 23 during application of a negative pressure. For example, the heater 14 may be 10 millimeters or less from the tobacco element 23. The heater 14 may be arranged to produce a temperature of 50 degrees Celsius at the mouth-end insert 8. Moreover, the heater 14 may heat the tobacco element 23 to a temperature between about 50 and about 200 degrees Celsius and heat the pre-vapor formulation at about 400 degrees Celsius.

The heater 14 warms the tobacco element 23, but does not burn the tobacco. Therefore, the warming of the tobacco element 23 may be referred to as non-combustible. Because the section 70 includes the tobacco element 23 and the heater 14, the section 70 may be referred to as a non-combustible smoking element.

Referring to FIG. 1, the wick 28, pre-vapor formulation supply reservoir 22 and mouth-end insert 8 are contained in the cartridge 70 and the power supply 1 is contained in the second section 72. In one embodiment, the first section (the cartridge) 70 is disposable and the second section (the fixture) 72 is reusable. The sections 70, 72 can be attached by a threaded connection 205, as described above, whereby the downstream section 70 can be replaced when the pre-vapor formulation supply reservoir 22 is used up. Having a separate first section 70 and second section 72 provides a number of advantages. First, if the first section 70 contains the at least one heater 14, the pre-vapor formulation supply reservoir 22 and the wick 28, all elements which are potentially in contact with the pre-vapor formulation are disposed of when the first section 70 is replaced. Therefore, there will be no cross-contamination between different mouth-end inserts 8, for example, when using different pre-vapor formulation materials. Also, if the first section 70 is replaced at suitable intervals, there is little chance of the heater becoming clogged with pre-vapor formulation. Optionally, the first section 70 and the second section 72 are arranged to lock together when engaged.

In an embodiment, the at least one air inlet 44 includes one or two air inlets 44, 44'. Alternatively, there may be three, four, five or more air inlets. If there is more than one air inlet 44, 44', the air inlets 44, 44' are located at different locations along the non-combustible smoking device 60. For example, as shown in FIG. 1, an air inlet 44a can be positioned at the upstream end of the non-combustible smoking device 60 adjacent a sensor 16 such that the sensor 16 supplies power to the heater 14 upon sensing an application of a negative pressure. Air inlet 44a should communicate with the mouth-end insert 8 so that a draw upon the mouth-end insert activates the sensor 16. The air from the air inlet 44a can then flow along the power supply 1 and to one or a combination of the central air passage 20 in the seal 15, other portions of the inner tube 62, and other portions of the outer tube 6. At least one additional air inlet 44, 44' can be located adjacent and upstream of the seal 15 or at any other desirable location. Altering the size and number of air inlets 44, 44' can also aid in establishing the resistance to draw of the non-combustible smoking device 60.

In an embodiment, the heater 14 is arranged to communicate with the wick 28 and to heat the pre-vapor formulation material contained in the wick 28 to a temperature sufficient to vaporize the pre-vapor formulation material and form a vapor.

The heater 14 may be a wire coil surrounding wick 28. Examples of suitable electrically resistive materials include titanium, zirconium, tantalum and metals from the platinum group. Examples of suitable metal alloys include stainless steel, nickel-, cobalt-, chromium-, aluminum- titanium- zirconium-, hafnium-, niobium-, molybdenum-, tantalum-, tungsten-, tin-, gallium-, manganese- and iron-containing alloys, and superalloys based on nickel, iron, cobalt, stainless steel. For example, the heater may be formed of nickel aluminides, a material with a layer of alumina on the surface, iron aluminides and other composite materials, the electrically resistive material may optionally be embedded in, encapsulated or coated with an insulating material or vice-versa, depending on the kinetics of energy transfer and the external physicochemical properties required. In one embodiment, the heater 14 comprises at least one material selected from the group consisting of stainless steel, copper, copper alloys, nickel-chromium alloys, superalloys and combinations thereof. In an embodiment, the heater 14 is formed of nickel-chromium alloys or iron- chromium alloys. In one embodiment, the heater 14 can be a ceramic heater having an electrically resistive layer on an outside surface thereof.

In another embodiment, the heater 14 may be constructed of an iron-aluminide (for example, FeAl or Fe.sub.3AI), such as those described in commonly owned U.S. Pat. No. 5,595,706 to Sikka et al. filed Dec. 29, 1994, or nickel aluminides (for example, Ni.sub.3AI). Use of iron-aluminides is particularly advantageous in that they exhibit high resistivity. FeAl exhibits a resistivity of approximately 180 micro-ohms, whereas stainless steel exhibits approximately 50 to 91 micro-ohms. The higher resistivity lowers current draw or load on the power source (battery) 1.

In one embodiment, the heater 14 comprises a wire coil which at least partially surrounds the wick 28. In that embodiment, the wire may be at least one of a metal wire and the heater coil that extends partially along the length of the wick 28. The heater coil may extend fully or partially around the circumference of the wick 28. In another embodiment, the heater coil is not in contact with the wick 28.

The heater 14 heats the pre-vapor formulation in the wick 28 by thermal conduction. Alternatively, heat from the heater 14 may be conducted to the pre-vapor formulation by means of a heat conductive element or the heater 14 may transfer heat to the incoming ambient air that is drawn through the non-combustible smoking device 60 during use, which in turn heats the pre-vapor formulation by convection.

In one embodiment, the wick comprises a ceramic material or ceramic fibers. As noted above, the wick 28 is at least partially surrounded by the heater 14. Moreover, in an embodiment, the wick 28 extends through opposed openings in the inner tube 62 such that end portions 29, 31 of the wick 28 are in contact with the pre-vapor formulation supply reservoir 22.

The wick 28 may comprise a plurality or bundle of filaments. In one embodiment, the filaments may be generally aligned in a direction transverse to the longitudinal direction of the non-combustible smoking device 60, but example embodiments are not limited to this orientation. In one embodiment, the structure of the wick 28 is formed of ceramic filaments capable of drawing the pre-vapor formulation via capillary action via interstitial spacing between the filaments to the heater 14. The wick 28 can include filaments having a cross-section which is generally cross-shaped, clover-shaped, Y-shaped or in any other suitable shape.

The wick 28 includes any suitable material or combination of materials. Examples of suitable materials are glass filaments and ceramic or graphite based materials. Moreover, the wick 28 may have any suitable capillarity to accommodate pre-vapor formulations having different physical properties such as density, viscosity, surface tension and vapor pressure. The capillary properties of the wick 28, combined with the properties of the pre-vapor formulation, ensure that the wick 28 is always wet in the area of the heater 14 to avoid overheating of the heater 14.

Instead of using a wick, the heater can be a porous material of sufficient capillarity and which incorporates a resistance heater formed of a material having a high electrical resistance capable of generating heat quickly.

In one embodiment, the wick 28 and the pre-vapor formulation storage medium 21 of the pre-vapor formulation supply reservoir 22 are constructed from an alumina ceramic. In another embodiment, the wick 28 includes glass fibers and the pre-vapor formulation storage medium 21 includes a cellulosic material or polyethylene terephthalate.

In an embodiment, the power supply 1 may include a battery arranged in the non-combustible smoking device 60 such that the anode is downstream of the cathode. An anode connector 4 contacts the downstream end of the battery. The heater 14 is connected to the battery by two spaced apart electrical leads.

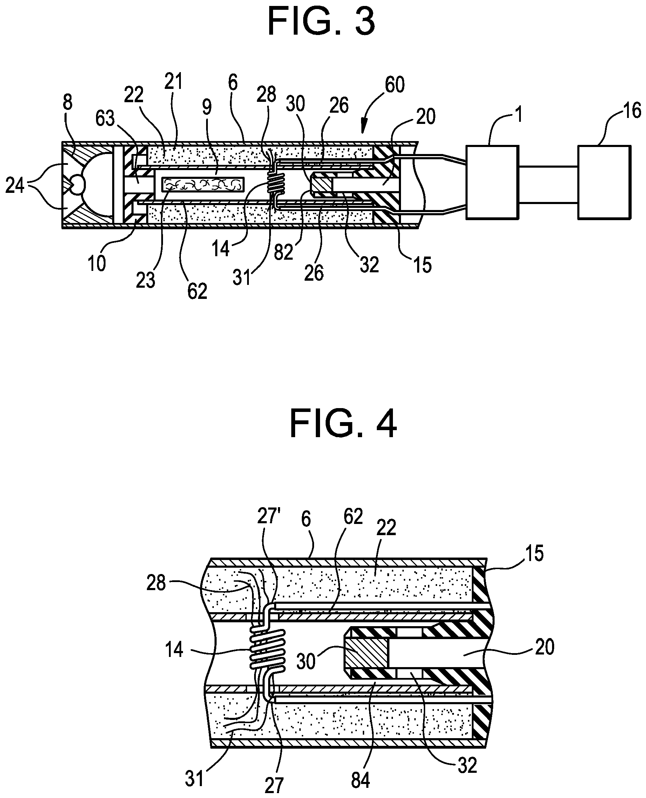

The connection between the uncoiled, end portions 27, 27' (see FIG. 4) of the heater 14 and the electrical leads are highly conductive and temperature resistant while the heater 14 is highly resistive so that heat generation occurs primarily along the heater 14 and not at the contacts.

The battery may be a Lithium-ion battery or one of its variants, for example a Lithium-ion polymer battery. Alternatively, the battery may be a Nickel-metal hydride battery, a Nickel cadmium battery, a Lithium- manganese battery, a Lithium-cobalt battery or a fuel cell. In that case, the non-combustible smoking device 60 is usable until the energy in the power supply is depleted. Alternatively, the power supply 1 may be rechargeable and include circuitry allowing the battery to be chargeable by an external charging device. In that case, the circuitry, when charged, provides power for a desired (or alternatively a pre-determined) number of applications of negative pressure, after which the circuitry must be re-connected to an external charging device.

The non-combustible smoking device 60 also includes control circuitry including the sensor 16. The sensor 16 is operable to sense an air pressure drop and initiate application of voltage from the power supply 1 to the heater 14. The control circuitry can also include a heater activation light 48 operable to glow when the heater 14 is activated. In one embodiment, the heater activation light 48 comprises a heater activation light (for example, a light emitting diode (LED)) 48 and is at an upstream end of the non-combustible smoking device 60 so that the heater activation light 48 takes on the appearance of a burning coal during an application of a negative pressure. Moreover, the heater activation light 48 can be arranged to be visible to the adult vaper. In addition, the heater activation light 48 can be utilized for e-vaping system diagnostics. The light 48 can also be configured such that the adult vaper can activate, deactivate, or activate and deactivate the light 48 for privacy, such that the light 48 would not activate during vaping if desired.

The at least one air inlet 44a is located adjacent the sensor 16, such that the sensor 16 senses air flow indicative of a negative pressure and activates the power supply 1 and the heater activation light 48 to indicate that the heater 14 is working.

A control circuit is integrated with the sensor 16 and supplies power to the heater 14 responsive to the sensor 16, for example, with a maximum, time-period limiter.

Alternatively, the control circuitry may include a manually operable switch for an application of a negative pressure. The time-period of the electric current supply to the heater 14 may be pre-set depending on the amount of pre-vapor formulation desired to be vaporized. The control circuitry may be programmable for this purpose. Alternatively, the circuitry may supply power to the heater as long as the sensor 16 detects a pressure drop.

When activated, the heater 14 heats a portion of the wick 28 surrounded by the heater for less than about 10 seconds, more preferably less than about 7 seconds. Therefore, the power cycle can range in period from about 2 seconds to about 10 seconds (for example, about 3 seconds to about 9 seconds, about 4 seconds to about 8 seconds or about 5 seconds to about 7 seconds).

In an embodiment, the pre-vapor formulation supply reservoir 22 includes the pre-vapor formulation storage medium 21 containing pre-vapor formulation material. In FIG. 1, the pre-vapor formulation supply reservoir 22 is contained in an outer annulus between inner tube 62 and outer tube 6 and between stopper 10 and the seal 15. Therefore, the pre-vapor formulation supply reservoir 22 at least partially surrounds the central air passage 20 and the heater 14 and the wick 28 extend between portions of the pre-vapor formulation supply reservoir 22.

The pre-vapor formulation storage medium 21 may be a fibrous material comprising at least one of cotton, polyethylene, polyester, rayon, and combinations thereof. The fibers may have a diameter ranging in size from about 6 microns to about 15 microns (for example, about 8 microns to about 12 microns or about 9 microns to about 11 microns). The pre-vapor formulation storage medium 21 may be a sintered, porous or foamed material. Also, the fibers may be sized to be irrespirable and can have a cross-section which has a y shape, cross shape, clover shape or any other suitable shape.

In another example embodiment, the pre-vapor formulation storage medium 21 may be a tobacco filler or tobacco slurry.

Also, the pre-vapor formulation material has a boiling point suitable for use in the non-combustible smoking device 60. If the boiling point is too high, the heater 14 will not be able to vaporize the pre-vapor formulation in the wick 28. However, if the boiling point is too low, the pre-vapor formulation may vaporize without the heater 14 being activated.

A pre-vapor formulation is a material or combination of materials that may be transformed into a vapor. For example, the pre-vapor formulation may be at least one of a liquid, solid or gel formulation including, but not limited to, water, beads, solvents, active ingredients, ethanol, plant extracts, natural or artificial flavors, vapor formers such as glycerine and propylene glycol, and combinations thereof.

The pre-vapor formulation may include a tobacco element including volatile tobacco flavor compounds which are released upon heating. When the tobacco element is in the pre-vapor formulation the physical integrity of the tobacco element is preserved. For example, the tobacco element may be between about 2% and about 30% by weight in the pre-vapor formulation.

For example, the tobacco element may be in the form of a sheet or shreds and is added after the pre-vapor formulation is added to the pre- vapor formulation storage medium 21.

In operation, with non-combustible smoking device 60 in an assembled configuration, a negative pressure may be applied on the mouth-end insert 8. This negative pressure may cause an internal pressure drop inside non-combustible smoking device 60 that may cause an inlet air flow to enter device 60 via air inlets 44 and 44'. The internal pressure drop may also cause an internal pressure drop within section 72 as air is drawn through air inlet 44a (via an air flow path traveling through section 72). The internal pressure drop formed in section 72 may be sensed by sensor 16. The sensor 16 may then operate to close an electrical circuit that includes the power supply 1. In turn, electrical leads carry an electrical current to heater 14 in order to energize the heater 14. The energized heater 14 in turn heats and vaporizes the pre-vapor formulation material that is drawn toward the heater 14 via the wick 28.

The pre-vapor formulation material is transferred from one or both of the pre-vapor formulation supply reservoir 22 and the pre-vapor formulation storage medium 21 in proximity of the heater 14 by capillary action in the wick 28. In one embodiment, the wick 28 has a first end portion 29 and a second opposite end portion 31 as shown in FIG. 3. The first end portion 29 and the second end portion 31 extend into opposite sides of the pre-vapor formulation storage medium 21 for contact with pre-vapor formulation material contained therein. The heater 14 at least partially surrounds a central portion of the wick 28 such that when the heater 14 is activated, the pre-vapor formulation in the central portion of the wick 28 is vaporized by the heater 14 to vaporize the pre-vapor formulation material and form the vapor. Due to a negative pressure being applied, the vapor flows from the heater 14, through the tobacco element 23 and out of the mouth-end insert 8.

The vapor may elute tobacco elements into the flow stream. Some thermal reactions may also be present between the vapor and the tobacco element.

One advantage of an embodiment is that the pre-vapor formulation material in the pre-vapor formulation supply reservoir 22 is protected from oxygen (because oxygen cannot generally enter the pre-vapor formulation storage portion via the wick) so that the risk of degradation of the pre-vapor formulation material is significantly reduced. Moreover, in some embodiments in which the outer tube 6 is not clear, the pre-vapor formulation supply reservoir 22 is protected from light so that the risk of degradation of the pre-vapor formulation material is significantly reduced. Thus, a high level of shelf-life and cleanliness can be maintained.

As shown in FIGS. 2A and 2B, the mouth-end insert 8, includes at least two diverging outlets 24 (for example, 3, 4, 5 or more). The outlets 24 of the mouth-end insert 8 are located at ends of off-axis passages 80 and are angled outwardly in relation to the longitudinal direction of the non-combustible smoking device 60 (that is, divergently). As used herein, the term "off-axis" denotes at an angle to the longitudinal direction of the non-combustible smoking device 60. Also, the mouth-end insert (or flow guide) 8 may include outlets uniformly distributed around the mouth-end insert 8 so as to substantially uniformly distribute the vapor during use. Therefore, the vapor moves in different directions as compared to e-vaping devices having an on-axis single orifice which directs the vapor to a single location.

In addition, the outlets 24 and off-axis passages 80 are arranged such that droplets of unvaporized pre-vapor formulation carried in the vapor impact interior surfaces 81 at one or both of the mouth-end insert and interior surfaces of the off-axis passages are removed or broken apart. In an embodiment, the outlets of the mouth-end insert are located at the ends of the off-axis passages and are angled at between about 5 and about 60 degrees with respect to the central axis of the outer tube 6 so as to more completely distribute vapor during use and to remove droplets.

Preferably, each outlet has a diameter of about 0.015 inch to about 0.090 inch (for example, about 0.020 inch to about 0.040 inch or about 0.028 inch to about 0.038 inch). The size of the outlets 24 and off-axis passages 80 along with the number of outlets can be selected to adjust the resistance to draw (RTD) of the non-combustible smoking device 60, if desired.

As shown in FIG. 1, an interior surface 81 of the mouth-end insert 8 can comprise a generally domed surface. Alternatively, as shown in FIG. 2B, the interior surface 81' of the mouth-end insert 8 can be generally cylindrical or frustoconical, with a planar end surface. The interior surface is substantially uniform over the surface thereof or symmetrical about the longitudinal axis of the mouth-end insert 8. However, in other embodiments, the interior surface can be irregular or have other shapes.

The mouth-end insert 8 is integrally affixed within the tube 6 of the section 70. Moreover, the mouth-end insert 8 may be formed of a polymer selected from the group consisting of low density polyethylene, high density polyethylene, polypropylene, polyvinylchloride, polyetheretherketone (PEEK) and combinations thereof. The mouth-end insert 8 may also be colored if desired.

In an embodiment, the non-combustible smoking device 60 also includes various embodiments of an air flow diverter or air flow diverter means. The air flow diverter is operable to manage air flow at or about the heater so as to abate a tendency of drawn air to cool the heater, which could otherwise lead to diminished vapor output.

In one embodiment, as shown in FIGS. 3-4, the non- combustible smoking device 60 can include an air flow diverter comprising an impervious plug 30 at a downstream end 82 of the central air passage 20 in seal 15. The central air passage 20 is an axially extending central passage in seal 15 and inner tube 62. The seal 15 seals the upstream end of the annulus between the outer and inner tubes 6, 62. The air flow diverter may include at least one radial air channel 32 directing air from the central air passage 20 outward toward the inner tube 62 and into the outer air passage 9 defined between an outer periphery of a downstream end portion of the seal 15 and the inner wall of inner tube 62.

The diameter of the bore of the central air passage 20 is substantially the same as the diameter of the at least one radial air channel 32. Also, the diameter of the bore of the central air passage 20 and the at least one radial air channel 32 may range from about 1.5 millimeters to about 3.5 millimeters (for example, about 2.0 millimeters to about 3.0 millimeters). Optionally, the diameter of the bore of the central air passage 20 and the at least one radial air channel 32 can be adjusted to control the resistance to draw of the non-combustible smoking device 60. In use, the air flows into the bore of the central air passage 20, through the at least one radial air channel 32 and into the outer air passage 9 such that a lesser portion of the air flow is directed at a central portion of the heater 14 so as to reduce or minimize the aforementioned cooling effect of the airflow on the heater 14 during heating cycles. Therefore, incoming air is directed away from the center of the heater 14 and the air velocity past the heater is reduced as compared to when the air flows through a central opening in the seal 15 oriented directly in line with a middle portion of the heater 14.

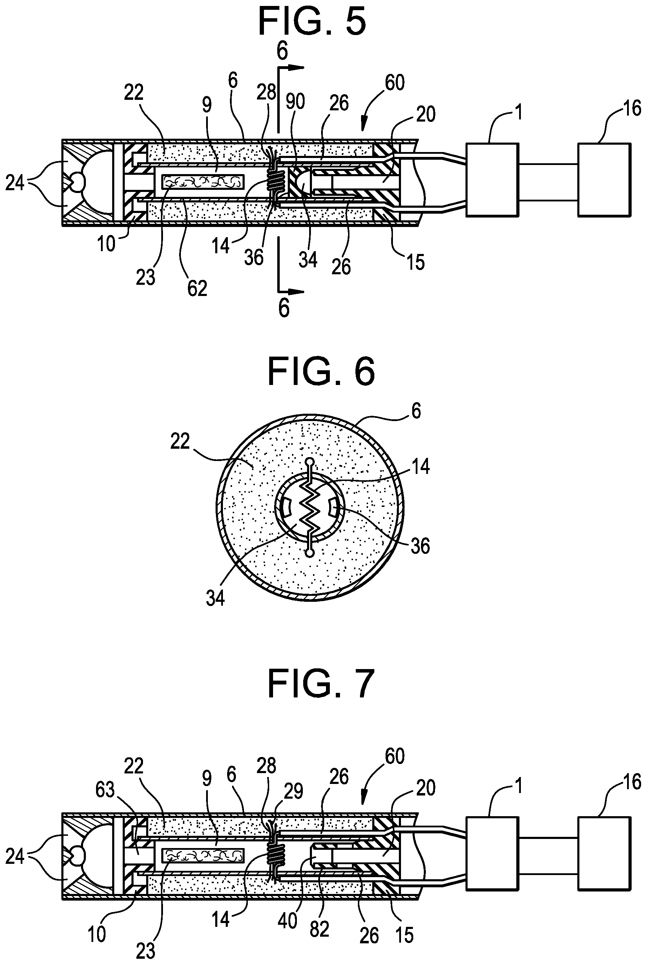

In another embodiment, as shown in FIGS. 5-6, the air flow diverter can be in the form of a disc 34 positioned between the downstream end of seal 15 and the heater 14. The disc 34 includes at least one orifice 36 in a transverse wall at a downstream end of an outer tubular wall 90. The at least one orifice 36 may be off-axis so as to direct incoming air outward towards the inner wall of tube 62. During an application of a negative pressure, the disc 34 is operable to divert air flow away from a central portion of the heater 14 so as to counteract the tendency of the airflow to cool the heater as a result of a strong or prolonged draw by an adult vaper. Therefore, the heater 14 is substantially reduced or prevented from cooling during heating cycles so as to reduce or prevent a drop in the amount of vapor produced during an application of a negative pressure.

In yet another embodiment, as shown in FIG. 7, the air flow diverter comprises a frustoconical section 40 extending from the downstream end 82 of a shortened central air passage 20. By shortening the central air passage 20 as compared to other embodiments, the heater 14 is positioned farther away from the central air passage 20 allowing the air flow to decelerate before contacting the heater 14 and lessen the tendency of the air flow to cool the heater 14. Alternatively, the heater 14 can be moved closer to the mouth-end insert 8 and farther away from the central air passage 20 to allow the air flow at least one of sufficient time and sufficient space to decelerate to achieve the same cooling-abatement effect.

The addition of the frustoconical section 40 provides a larger diameter bore size which can decelerate the air flow so that the air velocity at or about the heater 14 is reduced so as to abate the cooling effect of the air on the heater 14 during negative pressure cycles. The diameter of the large (exit) end of the frustoconical section 40 ranges from about 2.0 millimeters to about 4.0 millimeters, and preferably about 2.5 millimeters to about 3.5 millimeters.

The diameter of the bore of the central air passage 20 and the diameter of at least one of the smaller and the larger end of the frustoconical section 40 can be adjusted to control the resistance to draw of the non-combustible smoking device 60.

The air flow diverter of the various embodiments channels the air flow by controlling the air flow velocity (at least one of its speed and the direction of the air flow). For example, the air flow diverter can direct air flow in a particular direction, control the speed of the air flow, or both. The air flow speed may be controlled by varying the cross sectional area of the air flow route. Air flow through a constricted section increases in speed while airflow through a wider section decreases speed.

The outer tube 6, the inner tube 62, or both, may be formed of any suitable material or combination of materials. Examples of suitable materials include metals, alloys, plastics or composite materials containing one or more of those materials, or thermoplastics that are suitable for food or pharmaceutical applications, for example polypropylene, polyetheretherketone (PEEK), ceramic, and polyethylene. In one embodiment, the material is light and non-brittle.

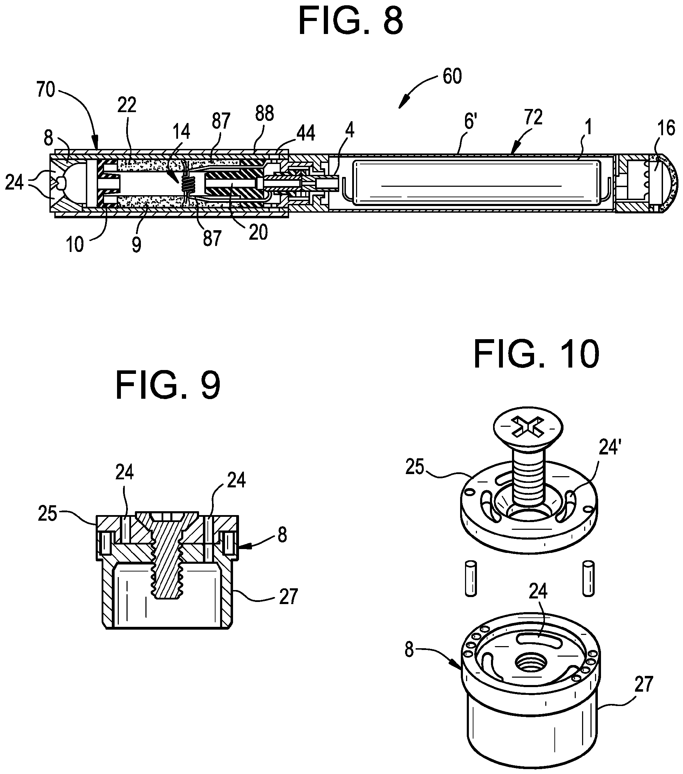

As shown in FIG. 8, the non-combustible smoking device 60 can also include a sleeve assembly 87 removably, rotatably, or removably and rotatably positioned about the outer tube 6 adjacent the first section 70 of the non-combustible smoking device 60. Moreover, the sleeve assembly 87 insulates at least a portion of the first section 70 so as to maintain the temperature of the vapor prior to delivery to the adult vaper. In an embodiment, the sleeve assembly 87 is rotatable about the non-combustible smoking device 60 and includes spaced apart slots 88 arranged transversely about the sleeve assembly such that the slots 88 line up with the air inlets 44 in the first section 70 to allow air to pass into the non-combustible smoking device 60 when a negative pressure is applied on the non-combustible smoking device 60. Before or during vaping, the adult vaper can rotate the sleeve assembly 87 such that the air inlets 44 are at least partially blocked by the sleeve assembly 87 so as to adjust at least one of the resistance to draw and the ventilation of the non-combustible smoking device 60.

The sleeve assembly 87 is made of silicone or other pliable material so as to provide a soft mouthfeel to the adult vaper. However, the sleeve assembly 87 may be formed in one or more pieces and can be formed of a variety of materials including plastics, metals and combinations thereof. In an embodiment, the sleeve assembly 87 is a single piece formed of silicone. The sleeve assembly 87 may be removed and reused with other non-combustible smoking devices or can be discarded along with the first section 70. The sleeve assembly 87 may be any suitable color, can include graphics or other indicia, or both.

As shown in FIGS. 9-10, in an alternative embodiment, the non-combustible smoking device can include a mouth-end insert 8 having a stationary piece 27 and a rotatable piece 25. Outlets 24, 24' are located in each of the stationary piece 27 and the rotatable piece 25. One or more of the outlets 24, 24' align as shown to allow vapor to enter an adult vaper's mouth. However, the rotatable piece 25 can be rotated within the mouth-end insert 8 so as to at least partially block one or more of the outlets 24 in the stationary piece 27. Thus, the amount of vapor output may be varied with each application of a negative pressure. The outlets 24, 24' can be formed in the mouth-end insert 8 such that the outlets 24, 24' diverge.

In another embodiment, the air flow diverter comprises the addition of a second wick element adjacent to but just upstream of the heater 14. The second wick element diverts portions of the airflow about the heater 14.

While FIGS. 1, 3, 5 and 7-8 illustrate a tobacco element in an outer air passage, example embodiments are not limited thereto.

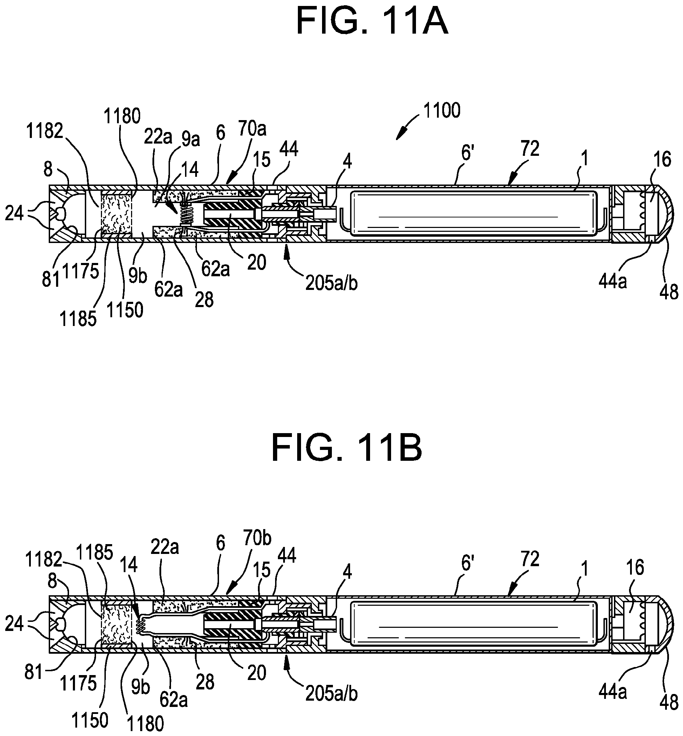

FIG. 11A illustrates an example embodiment of a non-combustible smoking device 1100 including a tobacco element 1150. The non-combustible smoking device 1100 is similar to the non-combustible smoking device 60. Thus, for the sake of brevity, only the differences will be described.

The non-combustible smoking device 1100 includes a pre-vapor formulation supply reservoir 22a. The pre-vapor formulation supply reservoir 22a is the same as the pre-vapor formulation supply reservoir 22 except the pre-vapor formulation supply reservoir 22a is shorter in the longitudinal direction.

A first section 70a includes the outer tube 6 (or housing) extending in a longitudinal direction and an inner tube 62a coaxially positioned within the outer tube or housing 6. The inner tube 62a defines a first outer air passage 9a. The first outer air passage 9a opens to a second outer air passage 9b.

An end of the inner tube 62a and the mouth-end insert 8 defines the second outer air passage 9b. In other words, the outer tube 6 may define a diameter in the latitudinal direction of the second outer air passage 9b. As shown, the diameter in the latitudinal direction of the second outer air passage 9b is larger than a diameter in the latitudinal direction of the first outer air passage 9a.

Within the second outer air passage 9b is the tobacco element 1150. The tobacco element 1150 may be inserted into the second outer air passage 9b by removing the mouth-end insert 8 and inserting the tobacco element 1150 into the second outer air passage 9b, for example.

The tobacco element 1150 may be a tobacco plug which refers to a compressed form of tobacco including, but not limited to tobacco strands, rolled tobacco or filler. The tobacco plug may be wrapped in natural tobacco, reconstituted sheet tobacco or aluminum, for example. While only one tobacco plug is illustrated, it should be understood that a plurality of tobacco plugs may be used. Fibrous segments (for example, cellulose acetate, other synthetic fibers, or natural fibers) may be placed between the plurality of tobacco plugs.

For example, a cylindrical housing 1185 holds tobacco. The cylindrical housing 1185 may be made of aluminum, for example. The cylindrical housing 1185 has an outer diameter that fits with the diameter of the outer air passage 9b. Along the longitudinal axis of the housing 6, mesh screens 1175 and 1180 fit at ends of the cylindrical housing 1185 to enclose the tobacco in the cylindrical housing 1185. As shown in FIG. 11A, the mesh screens 1175 and 1180 include openings 1182 to allow air to pass from one end of the cylindrical housing through the tobacco and out of the end of the cylindrical housing 1185 closest to the mouth-end insert 8.

The tobacco element 1150 is arranged in such a way to allow the vapor generated by the heater 14 to pass through the tobacco. For example, the tobacco element 1150 may be spaced a first distance from the mouth-end insert 8 and a second distance from the pre-vapor formulation supply reservoir 22. The first distance and the second distance may be the same or different.

Due to a negative pressure being applied, the vapor flows from the heater 14, through the tobacco element 1150 and out of the mouth-end insert 8. The heater 14 may be a set distance from the tobacco element 1150 or contacting the tobacco element 1150 such that the heater 14 heats the tobacco to a temperature (as described above) during an application of a negative pressure. In an example, the heater 14 may be between about 1 millimeter and about 5 millimeters from the tobacco element 1150.

While the inner tube 62a is shown as extending past the heater 14 in the longitudinal direction to the mouth-end insert 8, it should be understood that the heater 14 may be arranged to extend into the second outer air passage 9b. As a result, the tobacco element 1150 may be spaced apart from the heater 14 or in contact with the heater 14, such as shown FIG. 11B. In FIG. 11B, the heater 14 is in the second outer passage 9b of a section 70b. Thus, pre-vapor formulation supply reservoir 11a, the heater 14 and the tobacco element 1150 are sequentially arranged.

While the gasket 10 is not illustrated, the non-combustible smoking device 11 may include the gasket 10.

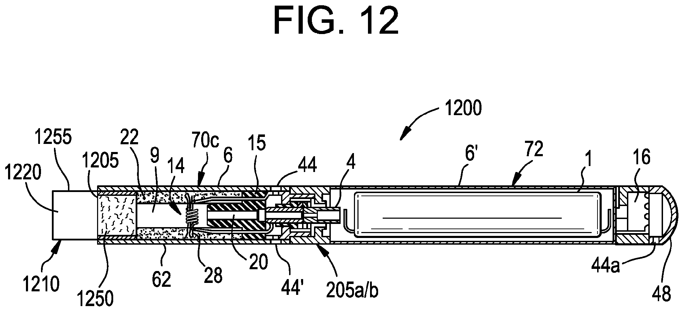

FIG. 12 illustrates an example embodiment of a non-combustible smoking device 1200. FIG. 12 illustrates an example embodiment of a non-combustible smoking device 1200 including a tobacco element 1250. The non-combustible smoking device 1200 is similar to the non-combustible smoking device 60 except a section 70c does not include the mouth-end insert 8, the tobacco element 23 and the gasket 10 and the non-combustible smoking device 1200 further includes an insert 1210. Therefore, for the sake of brevity, only the differences will be described.

By removing the mouth-end insert 8 and the gasket 10, the non-combustible smoking device 1200 includes a receiving area 1205 fitted to receive a tobacco insert 1210. The receiving area 1205 is defined by the outer tube 6 and an end of the pre-vapor formulation supply reservoir 22.

The tobacco insert 1210 may be a cigarette or cigar. For example, the tobacco insert may be a filtered cigarette, a non-filtered cigarette, a cigarillo, a filter tipped cigar filter, a tipped cigar or an untipped cigar or cigarillo. However, example embodiments are not limited thereto.

The tobacco insert 1210 is a detachable insert. In the example shown in FIG. 12, the tobacco insert 1210 may be a cigarette or a portion of a cigarette. The tobacco insert 1210 includes a filter 1220 and a tobacco element 1250. In example embodiments where the tobacco insert is an untipped cigar or cigarillo, the tobacco insert does not include a filter.

Tipping paper 1255 may overlap the filter 1220 and the tobacco element 1250. The tipping paper 1255 may cover surface areas of the tobacco insert 1210 that extend in along the outer tube 6. Therefore, the tipping paper 1255 provides stiffness to the tobacco insert 1210, permitting easier insertion to the receiving area 1205. An aluminum foil may also be used to contain the tobacco element 1250, with or without additional tipping paper.

The position of the heater 14 is not limited to the position shown in FIG. 12A. For example, the heater 14 may be positioned at the end of the outer air passage 9 such that the heater 14 is closer to the tobacco element 1250 or in contact with the tobacco element 1250. In another example embodiment, the heater 14 may protrude out of the outer air passage 9 in the same manner as shown in FIG. 11B.

The heater 14 may be a set distance from the tobacco element 1250 or contacting the tobacco element 1250 such that the heater 14 heats the tobacco element 1250 to a temperature (as described above) during an application of a negative pressure.

In operation, with non-combustible smoking device 1200 in an assembled configuration, a negative pressure may be applied on the tobacco insert 1210. The negative pressure may cause an internal pressure drop inside non-combustible smoking device 1200 that may cause an inlet air flow to enter the device 1200 via air inlets 44 and 44'. The internal pressure drop may also cause an internal pressure drop within section 72 as air is drawn through air inlet 44a (via an air flow path traveling through section 72). The internal pressure drop formed in section 72 may be sensed by sensor 16. The sensor 16 may then operate to close an electrical circuit that includes the power supply 1. In turn, electrical leads carry an electrical current to heater 14 in order to energize the heater 14. The energized heater 14 in turn heats and vaporizes a portion of the pre-vapor formulation that is drawn toward the heater 14 via the wick 28.

Pre-vapor formulation material is transferred from at least one of the pre-vapor formulation supply reservoir 22 and the pre-vapor formulation storage medium 21 in proximity of the heater 14 by capillary action in the wick 28. When the heater 14 is activated, the pre-vapor formulation in the central portion of the wick 28 is vaporized by the heater 14 to vaporize the pre-vapor formulation material and form vapor. Due to a negative pressure being applied, the vapor flows from the heater 14, through the tobacco element 1250 and out of the filter 1220.

In the example shown in FIG. 12, the filter 1220 may be a cellulose acetate (CA) filter. CA filter elements, such as triacetin, can be eluted into vapor. Vapor phase nicotine and other volatile elements in vapor can be reduced by a presence of tobacco.

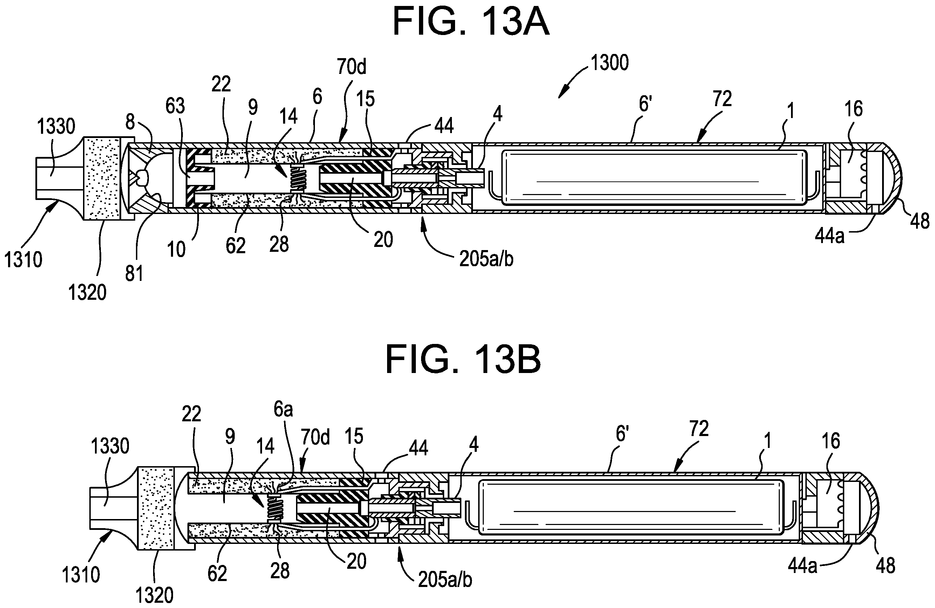

FIG. 13A illustrates an example embodiment of a non-combustible smoking device 1300.

The non-combustible smoking device 1300 is similar to the non-combustible smoking device 60 except a section 70d does not include the tobacco element 23 and the non-combustible smoking device 1300 further includes a detachable mouthpiece 1310. Therefore, for the sake of brevity, only the differences will be described.

The detachable mouthpiece 1310 includes a tobacco element 1320. The tobacco element 1320 may be contained in a plug or bag, and attached to the inside of mouthpiece 1310. The detachable mouthpiece 1310 fits over a portion the outer tube 6 to form a seal between the detachable mouthpiece and the section 70d. The detachable mouthpiece 1310 may form the seal by sliding onto the outer tube 6 or having a connection mechanism (for example, a male or female connection) to connect to the outer tube 6.

In operation, with non-combustible smoking device 1300 in an assembled configuration, a negative pressure may be applied on the detachable mouthpiece 1310. Due to a negative pressure being applied, the vapor flows from the heater 14, through the mouth-end insert 8, into the tobacco element 1320 and out of the detachable mouthpiece 1310 through an air passage 1330.

The heater 14 may be a set distance from the tobacco element 1320 or contacting the tobacco element 1320 such that the heater 14 heats the tobacco element 1320 to a temperature (as described above) during an application of a negative pressure.

In another example embodiment, the mouth-end insert 8 and the gasket 10 may be omitted such as shown in FIG. 13B. In the embodiment shown in FIG. 13B, a tube 6a is shorter than the tube 6, of FIG.13A.

In other example embodiments, the tobacco element may be in the pre-vapor formulation supply reservoir, the tobacco element may function as the pre-vapor formulation storage medium, or both.

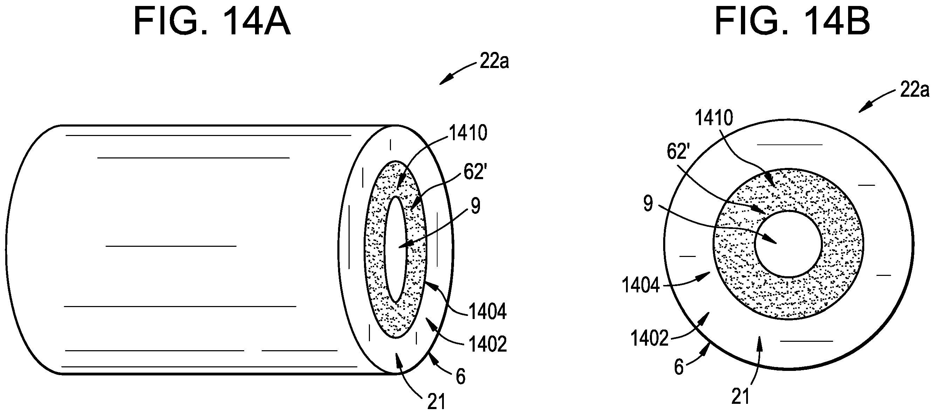

For example, FIGS. 14A-B illustrate an example embodiment of a pre-vapor formulation supply reservoir. A pre-vapor formulation supply reservoir 22a may be used as the pre-vapor formulation supply reservoir 22.

As shown, the pre-vapor formulation supply reservoir 22a includes a pre-vapor formulation 1402, an intermediate tube 1404, a tobacco element 1410 and an inner tube 62'. The inner tube 62' defines the air passage 9 and may include a metal grid, screen or mesh, for example.

In another example embodiment, the inner tube 62' may be formed of any suitable material or combination of materials. Examples of suitable materials include metals, alloys, plastics or composite materials containing one or more of those materials, or thermoplastics that are suitable for food or pharmaceutical applications, for example polypropylene, polyetheretherketone (PEEK), ceramic, and polyethylene.

The intermediate tube 1404 may include a glass fiber. The pre-vapor formulation 1402 is between the intermediate tube 1404 and the outer tube 6 and may be in the pre-vapor formulation storage medium 21.

The tobacco element 1410 is between the inner tube 62' and the intermediate tube 1404. The tobacco element 1410 may be tobacco sheet, shreds, powder, beads or a sponge, for example. The inner tube 62' may include extenders protruding into the tobacco to help heat transfer.

In operation, a negative pressure may be applied to the non-combustible smoking device, which activates the heater 14, as described above. The heater heats the pre-vapor formulation 1402 to form a vapor and the vapor flows from the heater 14, through the tobacco element 1410 and into the air passage 9.

As a result, the tobacco element 1410 is exposed to heat from the vapor and from the heater 14. Therefore, a tobacco aroma is imparted on the vapor.

In an example embodiment, an amount of tobacco element (for example, filler) in the non-combustible smoking device may produce about a same number of applications of a negative pressure as a cigarette. Alternatively, the amount of tobacco element may produce a fixed number of applications of a negative pressure.

In an example embodiment, the tobacco element may have nicotine removed.

Example embodiments described in FIGS. 1-14B may be combined to utilize a tobacco element in more than one location. For example, a first tobacco element can be combined with the pre-vapor formulation in the pre-vapor formulation supply reservoir and a second tobacco element may be in the passage 9. In other example embodiments, a first tobacco element can be combined with the pre-vapor formulation in the pre-vapor formulation supply reservoir and a second tobacco element may be a tobacco plug in the second outer air passage 9b. In another example embodiment, a first tobacco element can be combined with the pre-vapor formulation in the pre-vapor formulation supply reservoir and a second tobacco element may be in an insert or detachable mouthpiece. In another example embodiment, a first tobacco element can be in the passage 9 and a second tobacco element may be in an insert or detachable mouthpiece.

Example embodiments provide a non-combustible smoking device having a heater that heats a pre-vapor formulation and may provide heat to a tobacco element. More specifically, the non-combustible smoke device according to example embodiments exposes a vapor to a tobacco element, exposes a pre-vapor formulation to a tobacco element, or both. When the tobacco element is in the pre-vapor formulation the physical integrity of the tobacco element is preserved.

In other example embodiments, a non-combustible smoke device can be a pod device or tank device that exposes a vapor to a tobacco element, exposes a pre-vapor formulation to a tobacco element, or both.

While a single heater is described with reference to FIGS. 1-14B, example embodiments may include a multiple heater non-combustible smoking device. A first heater may be the heater 14 to vaporize the pre-vapor formulation and a second heater may be used to heat the tobacco element. The second heater may penetrate the tobacco element.

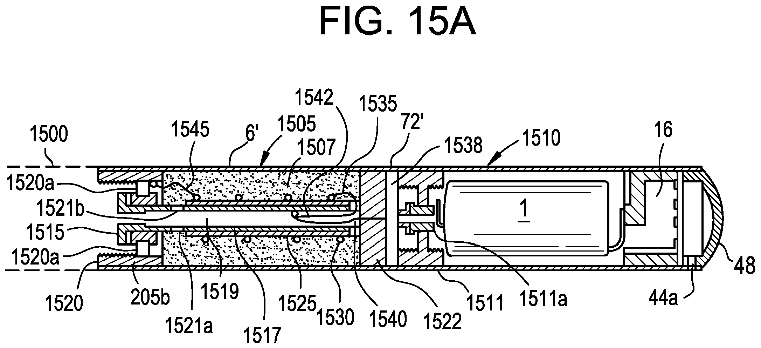

For example, FIGS. 15A-15B illustrates an example embodiment of a non-combustible smoking device having a plurality heaters.

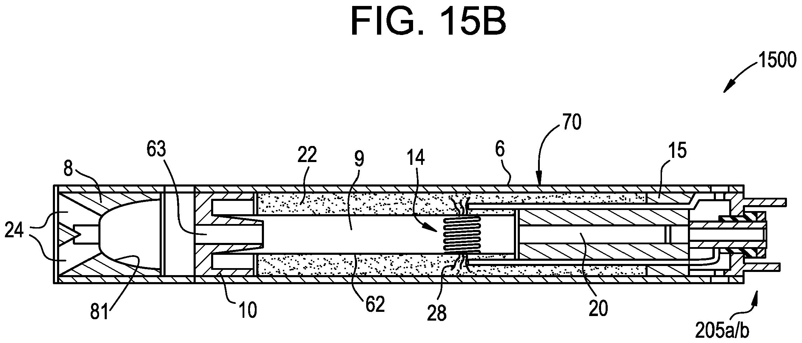

In FIG. 15A, a first section 1500 may be similar to the first section 70, shown in FIG. 1, without the tobacco element 23. FIG. 15B illustrates the first section 1500. Since the first section 1500 is the same as the first section 70 without the tobacco element 23, for sake of brevity, the first section 1500 is not described in further detail.

As shown in FIG. 15A, a second section 72' of the non- combustible smoking device includes a tobacco housing 1505 and a power housing 1510. The tobacco housing 1505 and the power housing 1510 may be separate cartridges that are connected together by a connecting portion 1511. The connecting portion 1511 may be the same as the threaded connection 205.

The tobacco housing 1505 houses tobacco 1507 and is configured to allow an aroma from the tobacco 1507 to flow into the first section 1500.

The tobacco housing includes the connector 205b, which has an anode portion 1515 and a cathode portion 1520. The anode portion 1515 includes an annular section 1517 that extends longitudinally in the tobacco housing 1505. The anode portion 1515 includes two holes 1521a and 1521b to allow air to flow into the tobacco 1507 and a channel 1519 when a negative pressure is applied on the mouth-end insert 8. Both the anode portion 1515 and the cathode portion 1520 include an electrically conductive material such as plated brass or stainless steel. The channel 1519 is defined in part by the anode portion 1515 in the longitudinal direction. A filter 1522 is located at one end of the channel 1519 and another end of the channel 1519 is open to the first section 1500. The filter 1522 may include cellulous acetate, glass fiber, ceramic, cotton, or any chemically inert porous material. As a result, the channel 1519 provides a path for air to flow into the tobacco 1507.

A fibrous sleeve 1525 covers at least a portion of the annular portion 1517 of the anode portion 1515. The fibrous sleeve 1525 may be a cellulosic material or polyethylene terephthalate and may extend from ends of the holes 1521a, 1521b to the filter 1522. The fibrous sleeve 1525 aids in controlling the temperature by absorbing heat emitted from a coiled heater 1530. The fibrous sleeve 1525 may be fiber glass or any material that is chemically inert and not electrically conductive. The fibrous sleeve 1525 electrically separates the heater 1530 and the anode portion 1515.

A coiled heater 1530 wraps around the fibrous sleeve 1525 in the longitudinal direction and heats the tobacco when power is supplied to the heater 1530 from the power supply 1. The heater 1530 may heat the tobacco and not burn it. For example, the heater 1530 may operate at around 190 degrees Celsius or could be varied based on a power supply control. The heater 1530 heats the tobacco 1507 to generate a tobacco aroma.

To receive power from the power supply 1, the heater 1530 is attached to the anode portion 1515 and the cathode portion 1520. More specifically, an anode of the power supply 1 is connected to an anode portion 1511a of the connecting portion 1511 which is connected to a battery connector 1538. The anode portion 1515 is connected to the battery connector by a wire 1540. While the wire 1540 is illustrated as passing through the filter 1540, the wire may pass between the filter 1540 and the outer tube 6'. The heater 1530 is connected to the anode portion 1515 by a wire 1535. The wire 1540 and 1535 form a soldered connection 1542 on the anode portion 1515.

In addition, the heater 1530 is soldered to wire 1545 which is connected to the cathode portion 1520. The wire 1545 may be connected to the cathode portion 1520 by, for example, spot welding or soldering the two electrical leads of the heater 252. It should be understood that connections should not be limited to soldering or spot welding. Where soldering is used welding may be used instead and vice versa.

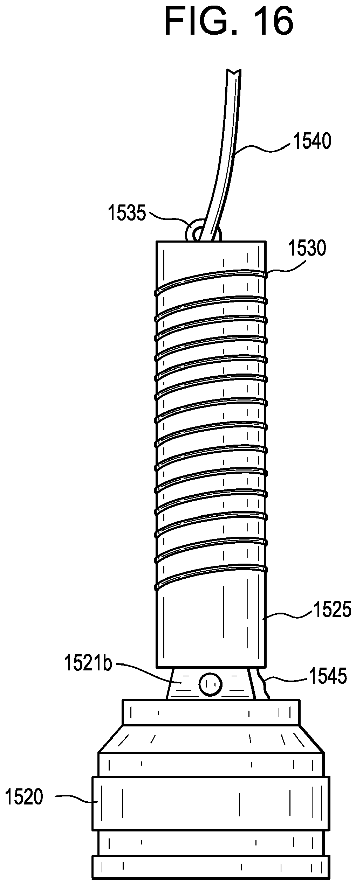

FIG. 16 illustrates a top view of the coiled heater 1530 surrounding the fibrous sleeve 1525. As shown, the coiled heater 1530 wraps around the fibrous sleeve 1525. The wire 1540 extends from the annular section 1517 of the anode portion 1515 past the fibrous sleeve 1525 to the battery connector 1538. Moreover, the sleeve 1525 extends to the hole 1521b of the anode portion 1515.

Referring back to FIG. 15A, the cathode portion 1520 includes holes 1520a.

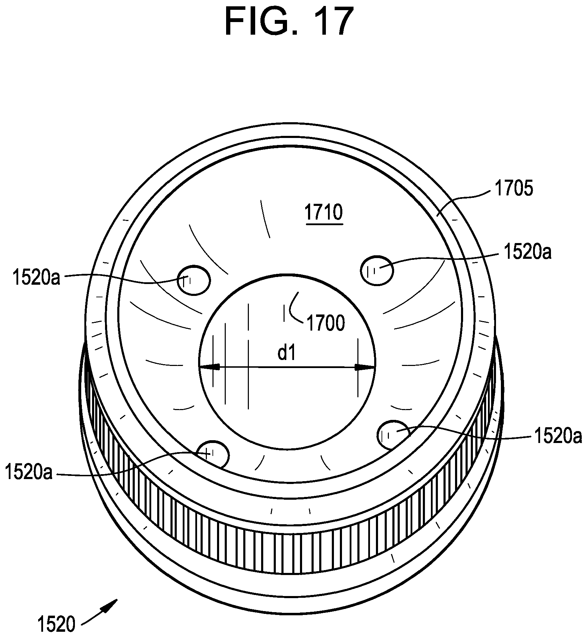

FIG. 17 illustrates a top view of the cathode portion 1520, according to an example embodiment. As shown, the cathode portion 1520 includes four holes 1520a. While four holes 1520a are illustrated, it should be understood that greater than or less than four holes may be used. Moreover, an inner surface 1700 has a diameter d1 that defines a receiving area for the anode portion.

The cathode portion 1520 includes an upper circular area 1705 and a lower circular area 1710. The holes 1520a are spaced approximately 90 degrees from each other and extend through the lower circular area 1710 to provide airways between the tobacco housing 1505 and the first section 1500.

More specifically, when a negative pressure is applied on the mouth-end insert 8, air flows through the channel 1519 as well as through the tobacco 1507 and the holes 1520a. The air flowing through the channel 1519 into the section 1500 will also have tobacco aroma due to the air flow path provided by the holes 1521a and 1521b in the anode portion 1515.

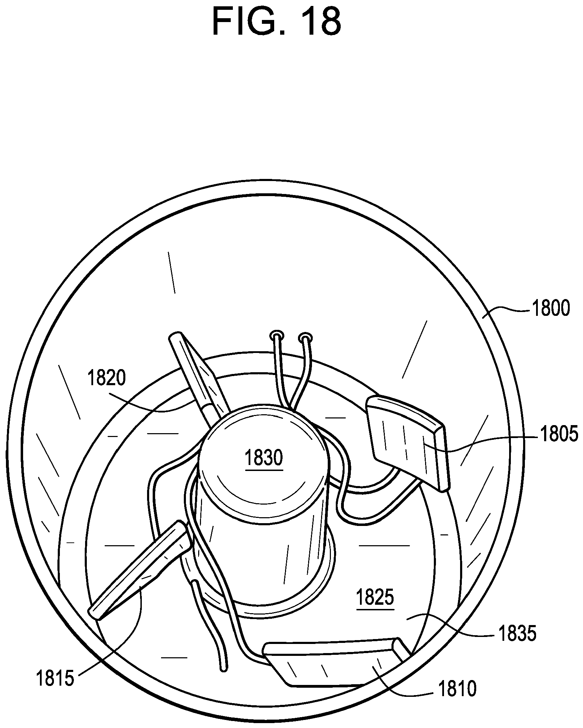

FIG. 18 illustrates a tobacco housing for a non-combustible smoking device according to an example embodiment. As shown in FIG. 18, a tobacco housing 1800 includes a tobacco receiving area 1825 and a protrusion 1830 extending from a surface 1835 of the tobacco receiving area 1825. The tobacco housing 1800 is cylindrical in shape and holds tobacco to be heated from heaters 1805, 1810, 1815 and 1820. The heaters 1805, 1810, 1815 and 1820 extend from the protrusion 1830 into the receiving area 1825. The tobacco housing 1800 may be upstream of a vapor generating area. Therefore, the heaters 1805, 1810, 1815 and 1820 heat the tobacco to provide an aroma to the vapor generated downstream. The heaters 1805, 1810, 1815 and 1820 are connected to a power source such as the power supply 1.

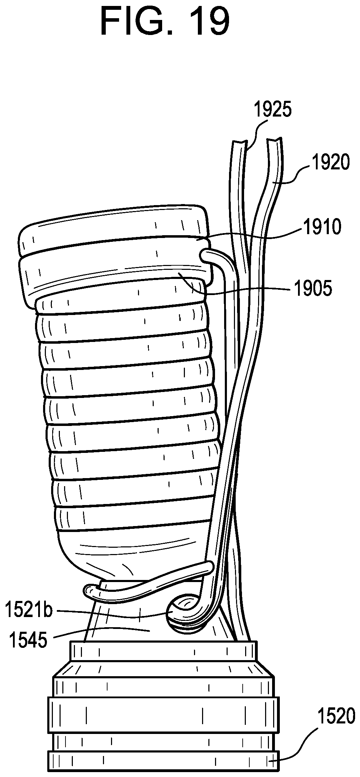

FIG. 19 illustrates another example embodiment of a non-combustible smoking device having a plurality heaters.

FIG. 19 illustrates a mesh heater 1905 covered in a fiber glass shield 1910 to help control the temperature. Tobacco is between the mesh heater 1905 and the fiber glass shield 1910. The mesh heater 1905 and fiber glass shield 1910 may be used instead of the tobacco heating arrangement illustrated in FIG. 15A. Therefore, the fiber glass shield 1910 may abut the housing 6. The mesh heater 1905 is connected to the power supply 1 through anode and cathode wires 1920 and 2915. The mesh is coiled from the top to the bottom of the cartridge.

The non-combustible smoking devices according to example embodiments may be stored in various configurations.

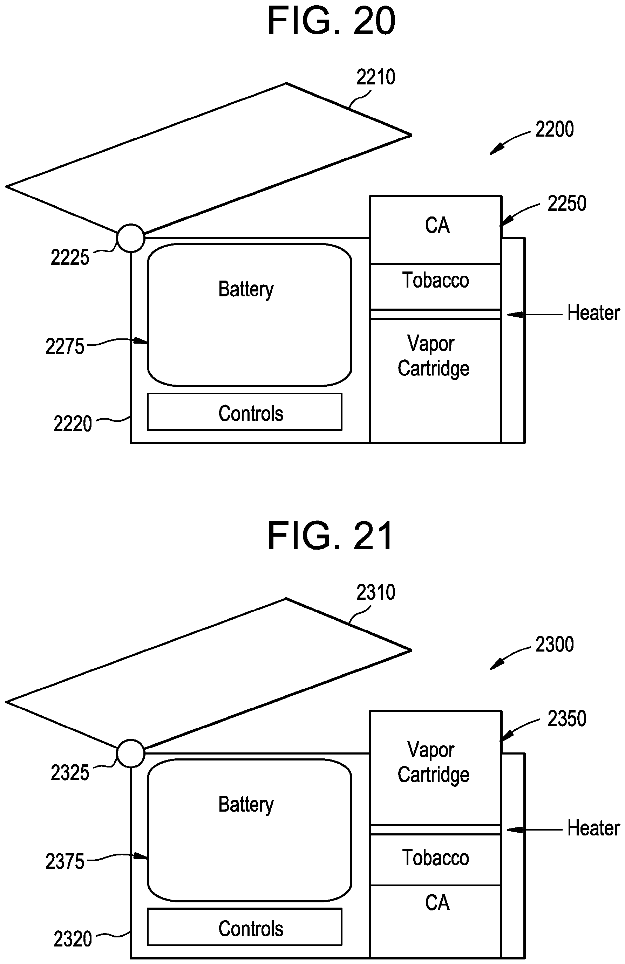

FIG. 20 illustrates a flip top container for a non-combustible smoking device according to an example embodiment.

As shown, a flip top container 2200 includes a top 2210 and a bottom receiving portion 2220. The bottom receiving portion 2220 is arranged in a fashion such that a first section 2250 of a non-combustible smoking device and a second section 2275 of the non-combustible smoking device are arranged side-by-side. For example, the first section 2250 may be the section 70c and the second section 2275 may be the section 72. The top portion 2210 may pivot about a hinge 2225, allowing an adult vaper to open and close the flip top container 2200.

FIG. 21 illustrates a flip top container for a non-combustible smoking device according to another example embodiment.

As shown, a flip top container 2300 includes a top 2310 and a bottom receiving portion 2320. The bottom receiving portion 2320 is arranged in a fashion such that a first section 2350 of a non-combustible smoking device and a second section 2375 of the non-combustible smoking device are arranged side-by-side. For example, the first section 2350 may be the section 70c and the second section 2375 may be the section 72. The top portion 2310 may pivot about a hinge 2325, allowing an adult vaper to open and close the flip top container 2300.

In other example embodiments, a non-combustible smoking device includes an inductive heater. The inductive heater may be the only heater in the non-combustible smoking device. The inductive heater may be provided in addition to a different type of electric heater, such as a resistive heater. The inductive heater may comprise an induction coil. The coil may be positioned outside of the tobacco. A reactive element, such as a susceptor, may be positioned on a surface of the tobacco, positioned within the tobacco, or both.

In other example embodiments, a temperature controller may be required to prevent over heating of the tobacco and prevent burning of the tobacco.

By utilizing one or a combination of a plurality of heaters, a coil heater, and a mesh heater, the surface area of tobacco exposed to heat increases thereby generating a larger amount of flavor to an adult vaper.

Example embodiments having therefore been described, it will be obvious that the same may be varied in many ways. Such variations are not to be regarded as a departure from the intended scope of example embodiments, and all such modifications as would be obvious to one skilled in the art are intended to be included within the scope of the following claims.

## Claims

1. A non-combustible smoking element comprising:
a pre-vapor formulation reservoir element (22) configured to contain a pre-vapor formulation material;
a pre-vapor heating element (14) coupled to the pre-vapor formulation reservoir element (22) and configured to heat at least a portion of the pre-vapor formulation material into a vapor and provide the vapor to a channel (1519);
a tobacco heating element (1530) configured to heat at least a portion of tobacco and generate an aroma; and
a tobacco housing (1505) configured to contain the tobacco and provide the aroma to the channel (1519),
wherein the tobacco housing (1505) includes:
an outer housing extending in a longitudinal direction;
an inner tube in the outer housing and extending in the longitudinal direction, a space between the outer housing and the inner tube defining a space to contain the tobacco; and
a connecting piece (205b) at a first end of the tobacco housing, the connecting piece including at least one first air inlet (1521a) to provide air to the space between the outer housing and the inner tube, and
wherein the connecting piece (205b) includes a second air inlet (1521b) to provide air within the inner tube.

2. The non-combustible smoking element of claim 1, wherein the tobacco heating element (1530) includes,
a plurality of heaters in the tobacco housing (1505).

3. The non-combustible smoking element of claim 2, wherein the plurality of heaters are upstream from the pre-vapor heating element (14).

4. The non-combustible smoking element of claim 2 or 3, wherein the plurality of heaters are outside the channel (1519) and the pre-vapor heating element (14) is in the channel (1519).

5. The non-combustible smoking element of any preceding claim, wherein the channel (1519) extends through the tobacco housing (1505).

6. The non-combustible smoking element of claim 1, wherein the tobacco heating element (1530) is a coil and extends around the inner tube.

7. The non-combustible smoking element of 6, wherein the tobacco heating element (1530) extends around the inner tube at an interval of between 1 and 2 millimeters.

8. The non-combustible smoking element of claim 1, wherein the tobacco housing (1505) includes,
one of a cellulose acetate (CA) filter, glass fiber filter, mesh screen and silicon gasket at a second end of the tobacco housing (1505).

9. The non-combustible smoking element of any preceding claim, wherein the tobacco heating element (1530) contacts the tobacco.

10. The non-combustible smoking element of any preceding claim, wherein the pre-vapor formulation reservoir element (22) and the tobacco housing (1505) are detachable.

11. The non-combustible smoking element of any preceding claim, wherein the tobacco is tobacco plant material in any form.

12. The non-combustible smoking element of any preceding claim, wherein the tobacco heating element (1530) is upstream from the pre-vapor heating element (14).

13. The non-combustible smoking element of any of claims 1 to 11, wherein the tobacco heating element (1530) is downstream from the pre-vapor heating element (14).

## Patentansprüche

1. Nicht brennbares Rauchelement, aufweisend:
ein Vordampfformulierungsvorratsbehälterelement (22), das derart ausgelegt ist, dass es ein Vordampfformulierungsmaterial enthält;
ein Vordampfheizelement (14), das mit dem Vordampfformulierungsvorratsbehälterelement (22) gekoppelt und ausgelegt ist, mindestens einen Teil des Vordampfformulierungsmaterials zu Dampf zu erwärmen und den Dampf einem Kanal (1519) bereitzustellen;
ein Tabakheizelement (1530), das ausgelegt ist, mindestens einen Teil des Tabaks zu erwärmen und ein Aroma zu erzeugen; und
ein Tabakgehäuse (1505), das ausgelegt ist, den Tabak zu enthalten und das Aroma an den Kanal (1519) bereitzustellen,
wobei das Tabakgehäuse (1505) einschließt:
ein Außengehäuse, das sich in einer Längsrichtung erstreckt;
ein Innenrohr in dem Außengehäuse, das sich in Längsrichtung erstreckt, wobei ein Raum zwischen dem Außengehäuse und dem Innenrohr einen Raum definiert, sodass er den Tabak enthält; und
ein Verbindungsstück (205b) an einem ersten Ende des Tabakgehäuses, wobei das Verbindungsstück mindestens einen ersten Lufteinlass (1521a) aufweist, um Luft an den Raum zwischen dem äußeren Gehäuse und dem inneren Rohr bereitzustellen, und
wobei das Verbindungsstück (205b) einen zweiten Lufteinlass (1521b) einschließt, um Luft innerhalb des Innenrohrs bereitzustellen.

2. Nicht brennbares Rauchelement nach Anspruch 1, wobei das Tabakheizelement (1530) einschließt,
mehrere Heizvorrichtungen in dem Tabakgehäuse (1505).

3. Nicht brennbares Rauchelement nach Anspruch 2, wobei sich die mehreren Heizelemente zuströmseitig von dem Vordampfheizelement (14) befinden.

4. Nicht brennbares Rauchelement nach Anspruch 2 oder 3, wobei sich die mehreren Heizelemente außerhalb des Kanals (1519) befinden und sich das Vordampfheizelement (14) in dem Kanal (1519) befindet.

5. Nicht brennbares Rauchelement nach einem der vorstehenden Ansprüche, wobei sich der Kanal (1519) durch das Tabakgehäuse (1505) erstreckt.

6. Nicht brennbares Rauchelement nach Anspruch 1, wobei das Tabakheizelement (1530) eine Spule ist und sich um das Innenrohr herum erstreckt.

7. Nicht brennbares Rauchelement nach Anspruch 6, wobei sich das Tabakheizelement (1530) in einem Abstand zwischen 1 und 2 Millimetern um das Innenrohr herum erstreckt.

8. Nicht brennbares Rauchelement nach Anspruch 1, wobei das Tabakgehäuse (1505) einschließt,
eines von einem Zelluloseacetat- (CA) -Filter, einem Glasfaserfilter, einem Maschensieb und einer Silikondichtung an einem zweiten Ende des Tabakgehäuses (1505).

9. Nicht brennbares Rauchelement nach einem der vorstehenden Ansprüche, wobei das Tabakheizelement (1530) den Tabak kontaktiert.

10. Nicht brennbares Rauchelement nach einem der vorstehenden Ansprüche, wobei das Vordampfformulierungsvorratsbehälterelement (22) und das Tabakgehäuse (1505) entfernbar sind.

11. Nicht brennbares Rauchelement nach einem der vorstehenden Ansprüche, wobei der Tabak Tabakpflanzenmaterial in irgendeiner Form ist.

12. Nicht brennbares Rauchelement nach einem der vorstehenden Ansprüche, wobei das Tabakheizelement (1530) zuströmseitig des Vordampfheizelements (14) angeordnet ist.

13. Nicht brennbares Rauchelement nach einem der Ansprüche 1 bis 11, wobei das Tabakheizelement (1530) nachgeschaltet des Vordampfheizelements (14) angeordnet ist.

## Revendications

1. Élément de fumage non combustible comprenant :
un élément de réservoir de la formulation pré-vapeur (22) configuré pour contenir un matériau de la formulation pré-vapeur ;
un élément de chauffage pré-vapeur (14) couplé à l'élément de réservoir de la formulation pré-vapeur (22) et configuré pour chauffer au moins une partie du matériau de la formulation pré-vapeur en une vapeur et fournir la vapeur à un canal (1519) ;
un élément de chauffage de tabac (1530) configuré pour chauffer au moins une partie du tabac et générer un arôme ; et
un logement de tabac (1505) configuré pour contenir le tabac et fournir l'arôme au canal (1519),
dans lequel le logement de tabac (1505) inclut :
un logement extérieur s'étendant dans une direction longitudinale ;
un tube intérieur dans le logement extérieur et s'étendant dans la direction longitudinale, un espace entre le logement extérieur et le tube intérieur définissant un espace pour contenir le tabac ; et
une pièce de raccordement (205b) à une première extrémité du logement du tabac, la pièce de raccordement incluant au moins une première entrée d'air (1521a) pour fournir de l'air à l'espace entre le logement extérieur et le tube intérieur, et
dans lequel la pièce de raccordement (205b) inclut une deuxième entrée d'air (1521b) pour fournir de l'air à l'intérieur du tube intérieur.

2. Élément de fumage non combustible selon la revendication 1, dans lequel l'élément de chauffage de tabac (1530) inclut,
une pluralité de dispositifs de chauffage dans le logement de tabac (1505).

3. Élément de fumage non combustible selon la revendication 2, dans lequel la pluralité de dispositifs chauffage sont en amont de l'élément de chauffage pré-vapeur (14).

4. Élément de fumage non combustible selon la revendication 2 ou 3, dans lequel la pluralité de dispositifs chauffage se trouvent à l'extérieur du canal (1519) et l'élément de chauffage pré-vapeur (14) se trouve dans le canal (1519).

5. Élément de fumage non combustible selon l'une quelconque des revendications précédentes, dans lequel le canal (1519) s'étend à travers le logement de tabac (1505).

6. Élément de fumage non combustible selon la revendication 1, dans lequel l'élément de chauffage de tabac (1530) est une bobine et s'étend autour du tube intérieur.

7. Élément de fumage non combustible selon la revendication 6, dans lequel l'élément de chauffage de tabac (1530) s'étend autour du tube intérieur à un intervalle compris entre 1 et 2 millimètres.

8. Élément de fumage non combustible selon la revendication 1, dans lequel le logement de tabac (1505) inclut,
l'un parmi un filtre en acétate de cellulose (CA), un filtre en fibre de verre, un tamis à mailles et un joint en silicone à une deuxième extrémité du logement de tabac (1505).

9. Élément de fumage non combustible selon l'une quelconque des revendications précédentes, dans lequel l'élément de chauffage de tabac (1530) entre en contact avec le tabac.

10. Élément de fumage non combustible selon l'une quelconque des revendications précédentes, dans lequel l'élément de réservoir de la formulation pré-vapeur (22) et le logement de tabac (1505) sont détachables.

11. Élément de fumage non combustible selon l'une quelconque des revendications précédentes, dans lequel le tabac est un matériau végétal de tabac sous n'importe quelle forme.

12. Élément de fumage non combustible selon l'une quelconque des revendications précédentes, dans lequel l'élément de chauffage de tabac (1530) est en amont de l'élément de chauffage pré-vapeur (14).

13. Élément de fumage non combustible selon l'une quelconque des revendications 1 à 11, dans lequel l'élément de chauffage de tabac (1530) est en aval de l'élément de chauffage pré-vapeur (14) .
